# EUROPEAN PATENT APPLICATION

(11) **EP 2 060 544 A1**
(43) Date of publication of application: **20.05.2009**
(21) Application number: 07022319.3
(22) Date of filing: 16.11.2007
(51) Int. Cl.: C02F 11/04

(54) **Method and apparatus for preparing material for microbiologic fermentation**

(71) Applicant: APV Systems Ltd., Bressenden Place London SW1E 5BF (GB)
(72) Inventor: Skudder, Paul John, Horsham West Sussex RH13 6PE (GB)
(74) Representative: Friese, Martin

(57) **Abstract**

The present invention relates to a method and an apparatus for preparing material for microbiological fermentations of municipal waste water sludge for reducing and/or cleaning the waste water sludge and/or of cellulosic material for production of biofuels, wherein a jet pump for disintegration or liquification of organic material is used.

## Description

The present invention relates to a method and an apparatus for preparing material for microbiological fermentations of organic and/or cellulosic material in accordance with the preamble of claim 1 and claim 7, respectively.

Microbiological fermentations are used in many processes for example, the aerobic and anaerobic digestion of municipal waste water and in the production of biofuels from cellulosic material.

The efficiency of the fermentation process is affected by the ability of the micro-organisms to access the organic matter due to the presence of aggregates and or solid material.

In an-aerobic digestion of waste water, a sludge forms in the bottom of the digester that must be removed, dried and disposed of at substantial cost. WO 01/16037 A1 discloses a method for mechanical disruption of the sludge using a homogeniser. A schematic diagram of this process is shown in Fig. 1. The whole disclosure of WO 01/16037 A1 is hereby incorporated by reference. The homogeniser disrupts the sludge to smaller aggregates that may be returned to the an-aerobic digester whereupon the bacteria can continue digestion. As a result additional methane gas is produced and the final volume of sludge that has to be removed is reduced.

The process is energy intensive in the electrical power required to operate the mechanical homogeniser but a net energy balance can be obtained by pre-concentration of the sludge by a minimum factor of 1.5 to reduce the volume of material passing through the homogeniser. The energy obtained by the conversion of the additional methane to electricity offsets the electrical requirements of the homogeniser.

WO 00/07946 A1 (EP 110276 A1; Applicant Paradigm) discloses the use of a mechanical homogeniser to homogenise sludge from the upstream aerobic digesters in waste water treatment prior to entry to the an-aerobic digesters. In this instance, very high homogenisation pressures are required and it is necessary to add sodium hydroxide to lyse the sludge to assist in the disruption process. A schematic diagram of this process is shown in Fig. 2. The whole disclosure of WO 00/07946 A1 is hereby incorporated by reference.

Apart from the high energy consumption, a problem in both of the above described processes is rapid damage to the homogenisation valves by sand and other abrasive material in the waste water. This necessitates frequent and costly maintenance and replacement of the homogeniser valves.

The object of the invention is to provide an apparatus and a method for microbiological fermentation in accordance with the preamble of the independent claims with an improved efficiency.

The object of the invention is achieved with the features of the independent claims. Preferred embodiments of the invention are disclosed in the dependent claims.

In the production of biofuels from cellulosic material, liquificaction will greatly improve the accessibility of the cellulose to subsequent acid or enzymatic hydrolysis and potentially release more fermentable sugars for conversion to alcohol.

The use of a jet pump as detailed in EP1549856B1 and or WO2006/010949 A1 can be used to substitute for the mechanical homogenisation of both aerobic and an-aerobic municipal waste water sludge with the benefit of reduced capital and operating cost whilst minimising mechanical damage resulting in longer operating times between maintenance of the disruption device. The whole disclosure of EP1549856B1 and WO2006/010949 A1 is hereby incorporated by reference.

The production of biofuel from sugar and wheat is well documented. However, diversion of food grade crops to biofuel will cause food prices to increase and a shortage of food. Other sources of fermentable substances must be found that would otherwise be considered a waste product. Such products include cellulosic material that once liquified and hydrolysed provides a source of fermentable sugar. Mechanical means of liquification are costly and equipment can be expensive in energy usage and in maintenance.

In order to be commercially successful, the costs of producing ethanol from cellulosic material must be less than oil. It is therefore advantageous to be able to utilize inexpensive and readily available sources of cellulosic material such as cut grass, wheat stalks, sugar cane waste and/or sugar beet waste products.

A jet pump as detailed in EP1549856B1 and or WO2006/010949 A1 can similarly be applied to liquify suspensions of cellulosic products in water such as cut grass and other products such as wheat stalks, sugar cane waste and sugar beet waste. Indeed, any cellulosic product. The resultant liquor may then be either enzymatically or chemically hydrolysed to fermentable sugars.

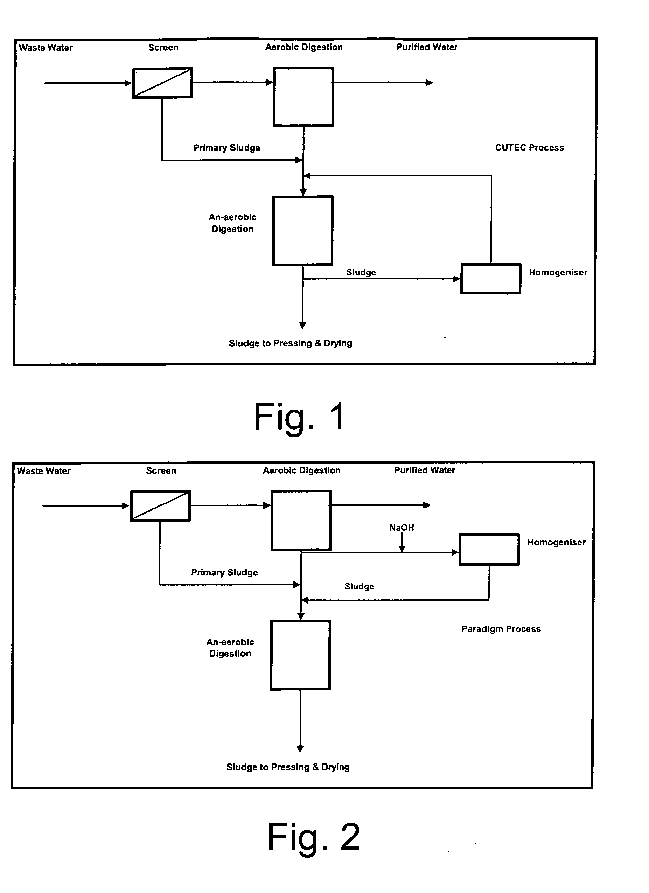

Fig. 1 is a schematic sketch of an apparatus and/or a method as disclosed in WO 01/16037 A1.

Fig. 2 is a schematic sketch of an apparatus and/or a method as disclosed in WO 00/07946 A1.

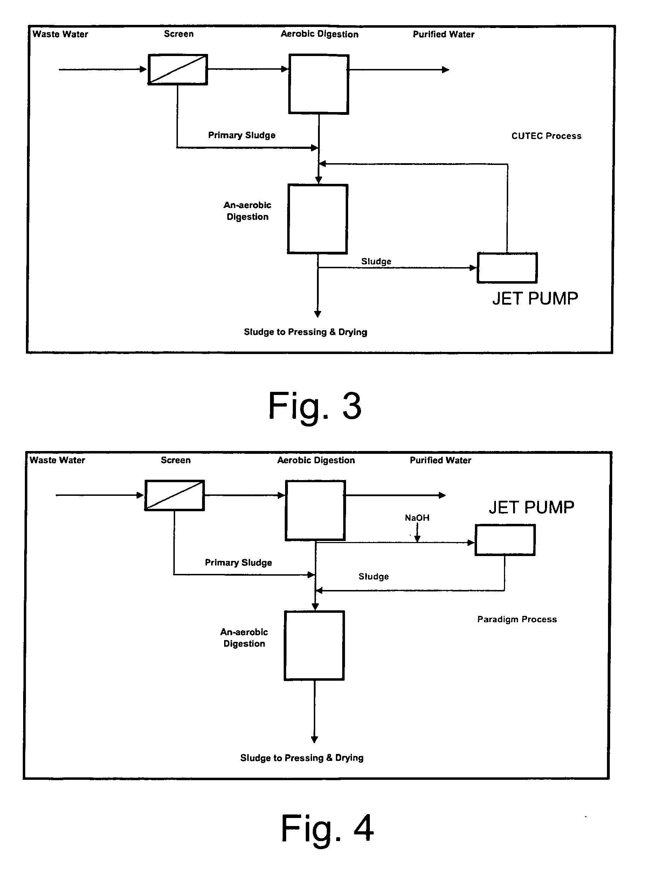

Fig. 3 is a schematic sketch of an apparatus and/or a method in accordance with a first embodiment of the invention.

Fig. 4 is a schematic sketch of an apparatus and/or a method in accordance with a second embodiment of the invention.

In accordance with the invention in addition and preferable before or better instead of the homogenizer as used in the prior art methods and apparatuses, a jet pump is used.

Accordingly a jet pump as known in the prior art as mentioned above and incorporated by reference is to be used instead of the homogenizer. In other applications the jet pump can be used in the process before the homogenizer.

Accordingly the invention also relates to a method and an apparatus for microbiologic fermentation of cellulosic material, wherein a jet pump for liquifying precut or pulverised cellulosic material is used.

## Claims

1. Method for preparing material for microbiological fermentations of municipal waste water sludge for reducing the waste water sludge and/or of cellulosic material for the production of biofuels,
**characterized by** using a jet pump for disintegration or liquification of organic material.

2. The method of claim 1, wherein the municipal waste water sludge is recirculated through the jet pump and returned to the aerobic or an-aerobic fermenter.

3. The method of claim 1, wherein the organic material comprises cellulosic material and wherein liquifying of the cellulosic material takes place before fermenting the cellulosic material.

4. The method of any of the preceeding claims, wherein a jet pump is used for liquification of a slurry in water of precut or pulverised cellulosic material.

5. The method of any of the preceding claims, wherein chemicals such as sodium hydroxide are added to aid the disruption or liquification process preferably before or during the disruption or liquification step.

6. The method of any of the preceding claims, wherein acid or acids are added to enable acid hydrolysis during disintegration or liquifaction.

7. The method of any of the preceding claims, wherein acid or acids are added post liquification to enable hydrolysis.

8. The method of any of the preceding claims, wherein enzymes are added to hydrolyse the cellulose.

9. The method of any of the preceding claims, wherein inexpensive and readily available sources of cellulosic materials are utilized, such as cut grass, wheat stalks, sugar cane waste and/or sugar beet waste products.

10. The method of any of the preceding claims, wherein biofuel is produced, for instance to be used as for heating devices and/or internal combustion engines of motor vehicles.

11. Apparatus for preparing material for microbiological fermentations of municipal waste water sludge for reducing the waste water sludge and/or of cellulosic material for production of biofuels
**characterized by** a jet pump.

12. The Apparatus of the preceding claim wherein the jet pump is adapted and arranged for liquifying pulverised cellulosic material and/or reducing waste water sludge.

13. The apparatus of any of the two preceding claims, being arranged and adapted for performing a method in accordance with any of the preceding method claims.

14. The method or apparatus of any of the preceding claims, wherein more than one jet pump is used or comprised, wherein the jet pumps are preferably used or connected in series.
